Europäisches Patentamt

European Patent Office

Office européen des brevets

(19)

(11) EP 0 781 996 A1

(12) ## DEMANDE DE BREVET EUROPEEN

(43) Date de publication:
02.07.1997 Bulletin 1997/27

(51) Int Cl.⁶: $G01N\ 33/28$

(21) Numéro de dépôt: 96402908.6

(22) Date de dépôt: 26.12.1996

(84) Etats contractants désignés:
BE DE ES IT NL SE

(30) Priorité: 28.12.1995 FR 9515672

(71) Demandeur: ELF ANTAR FRANCE
92400 Courbevoie (FR)

(72) Inventeurs:
• Puel, Cécile
69540 Irigny (FR)

• Hartmann, Francois
69002 Lyon (FR)
• Saby, Claude Alain
69500 Bron (FR)

(74) Mandataire: Ohresser, François
Elf Aquitaine Production,
Département Propriété Industrielle,
Tour Elf
92078 Paris La Défense Cedex (FR)

(54) **Procédé de détermination de la valeur d'une grandeur physique**

(57) Procédé de détermination de la valeur d'une grandeur physique d'un produit à analyser à partir des valeurs d'enregistrements de signaux délivrés par au moins un instrument de mesure alimenté par ledit produit et d'un modèle validé, construit à partir d'un fichier initial de données numériques, consistant en outre à repartir les données numériques du fichier initial en sous-groupes homogènes et représentatifs, entre eux et par rapport audit fichier initial.

Le procédé de l'invention trouve son application notamment dans les industries pétrolières, chimiques et agroalimentaires.

EP 0 781 996 A1

**Description**

DOMAINE TECHNIQUE

La présente invention concerne un procédé de détermination de la valeur d'une grandeur physique d'un produit à analyser à partir des enregistrements des valeurs délivrées par au moins un instrument de mesure alimenté par ledit produit et d'un modèle mathématique validé.

Elle trouve son application dans les laboratoires de contrôle, les laboratoires de recherche, les unités de production et les unités de fabrication des industries pétrolières, chimiques, pétrochimiques, pharmaceutiques, cosmétologiques et agroalimentaires.

ETAT DE LA TECHNIQUE ANTERIEURE

Une méthode connue de détermination d'une grandeur physique d'un produit à analyser consiste à exécuter les étapes suivantes :

- enregistrer les données délivrées par un instrument de mesure, par exemple un spectromètre, alimenté successivement par une pluralité de produits dont la valeur de la grandeur physique est connue. Tous ces enregistrements étant considérés comme un ensemble d'observations, constituent un fichier de données initiales,
- construire, à partir d'un fichier d'apprentissage extrait du fichier initial, un modèle qui établit une relation mathématique entre les données délivrées par l'appareil de mesure et la grandeur physique,
- valider le modèle à partir d'un fichier test extrait du fichier initial,
- appliquer le modèle aux données délivrées par l'instrument de mesure alimenté par un produit dont on veut déterminer la grandeur physique.

La qualité de ce modèle dépend du degré d'homogénéité et de représentativité du fichier test vis-à-vis du fichier d'apprentissage. Une méthode pour améliorer la qualité du modèle, lors de l'étape de répartition du fichier initial en deux sous-ensembles : apprentissage et test, est connue sous le nom d'algorithme de "KENNARD et STONE". Elle est décrite dans un article de la revue scientifique TECHNOMETRICS 11 (1969), 137.

Selon cette méthode, toutes les observations sont considérées comme étant candidates au fichier test. Les observations sont choisies séquentiellement, de telle sorte que la répartition ainsi créée soit uniforme dans l'espace des variables.

Les deux observations les plus éloignées sont sélectionnées en premier. Les suivantes sont choisies les unes après les autres comme les plus proches des observations précédemment sélectionnées, au sens de la distance euclidienne.

Ainsi trois quarts des observations sont sélectionnés pour former un fichier d'apprentissage, le reste constituant le fichier test.

Cette méthode a comme caractéristique de privilégier les données extrêmes et d'être sensible aux observations particulières, ce qui a pour effet de fausser les valeurs de la grandeur physique évaluée par le modèle. Cet inconvénient est particulièrement inacceptable lorsque la grandeur à déterminer est utilisée pour conduire une unité de fabrication du produit.

EXPOSE DE L'INVENTION

La présente invention a justement pour objet de remédier à ces inconvénients et de fournir des connaissances sur la densité, la forme et l'orientation des sous-groupes d'observations, qui déterminent la qualité de la modélisation.

De plus, l'invention permet d'optimiser la répartition selon une stratégie basée sur cette qualité.

A ces fins, l'invention propose un procédé de détermination de la valeur d'une grandeur physique d'un produit à analyser à partir des valeurs d'enregistrements des signaux délivrés par au moins un instrument de mesure alimenté par ledit produit et d'un modèle validé, construit à partir d'un fichier initial de données numériques préalablement obtenues à partir des valeurs des enregistrements des signaux délivrés par l'instrument de mesure alimenté par une pluralité de produits et des valeurs connues de la grandeur physique de chacun desdits produits, consistant en outre à répartir les données numériques du fichier initial en sous-groupes homogènes et représentatifs, entre eux et par rapport audit fichier initial.

Selon une autre caractéristique le procédé de l'invention comporte les étapes consistant à :

- soumettre les données numériques du fichier initial à une transformation mathématique,
- choisir un nombre de sous-groupes supérieur ou égal à deux,

- choisir la taille de chacun desdits sous-groupes,
- générer une ou plusieurs répartitions initiales des données numériques dans lesdits sous-groupes,
- évaluer la qualité de la ou des répartitions initiales des données numériques en sous-groupes à partir d'au moins un critère de qualité,
- choisir la répartition à modifier dans l'étape suivante dans le cas d'une pluralité de répartitions initiales,
- générer une nouvelle répartition par modification de la répartition des données numériques dans les sous-groupes selon une technique d'optimisation,
- évaluer la qualité de la nouvelle répartition à partir du ou des critères de qualité,
- réitérer les trois étapes précédentes jusqu'à satisfaction d'un critère d'arrêt obtenu par comparaison de la valeur de la qualité de la dernière répartition à un seuil fixé ou jusqu'à ce que le nombre d'itérations atteigne un nombre maximal fixé.

Selon une autre caractéristique de l'invention, le critère de qualité consiste en au moins un critère choisi parmi les suivants :

- critère de représentativité des données numériques des sous-groupes par rapport à la grandeur mesurée, qui met en oeuvre une métrique caractérisant une distance entre les sous-groupes,
- critère d'homogénéité servant à comparer entre elles d'une part les formes et d'autre part les orientations des sous-groupes,
- critère d'homogénéité servant à comparer entre elles les densités des données numériques dans les sous-groupes.

Selon une autre caractéristique de l'invention, la technique d'optimisation consiste en au moins une méthode choisie parmi les suivantes :

- méthode qui met en oeuvre au moins un algorithme génétique,
- méthode qui met en oeuvre au moins un réseau de neurones,
- méthode qui met en oeuvre au moins un algorithme de recuit simulé,
- méthode incrémentale,
- méthode qui met en oeuvre au moins un critère matriciel d'optimalité, et
- méthode itérative de type bootstrap.

Selon une autre caractéristique du procédé le choix de la taille des sous-groupes est optimisé par application d'une méthode d'optimisation itérative d'un critère de qualité.

## DESCRIPTION DETAILLEE DE L'INVENTION

D'une manière générale le procédé de l'invention est utilisé pour déterminer la valeur d'une grandeur physique d'un produit à partir des valeurs d'enregistrements de signaux délivrés par au moins un instrument de mesure.

Selon un mode particulier d'utilisation, le procédé de l'invention sert à déterminer, l'indice d'octane moteur d'une essence issue d'une unité de craquage catalytique dans une raffinerie de pétrole brut. Selon ce mode particulier de réalisation on enregistre pendant plusieurs jours les valeurs de six variables, représentatives des conditions opératoires du craqueur catalytique et de données analytiques caractérisant la charge, à savoir :

- la température de réaction,
- la température de préchauffe,
- la teneur en soufre,
- la teneur en métaux lourds,
- le % de résidu atmosphérique,
- le rapport C/H.

Les six variables relevées un jour, constituent une observation. Pour chaque observation la valeur de l'indice d'octane de l'essence produite, déterminée par une mesure directe en laboratoire, est enregistrée.

Le fichier initial contient les enregistrements de 144 observations et les valeurs correspondantes de l'indice d'octane de l'essence produite.

L'étape suivante consiste à centrer et réduire chacune des variables pour les normaliser.

Ensuite on choisit un nombre de sous-groupes égal à 3, pour constituer un sous-groupe d'apprentissage, un sous-groupe de sélection et un sous-groupe de validation.

Afin d'obtenir une précision suffisante pour la validité du modèle, on fixe la taille des sous-groupes de sélection et de validation respectivement à 25 et 24 observations, tout en conservant suffisamment d'observations pour le sous-groupe d'apprentissage, soit 95 dans notre exemple.

On génère un ensemble de 20 répartitions initiales des données du fichier initial puis on évalue la qualité de ces 20 répartitions initiales au moyen d'un critère global de qualité F, fonction de deux critères d'homogénéité P et C, et d'un critère de représentativité R.

Le critère R définit une distance entre deux sous-groupes selon la métrique de MAHALANOBIS. Il est calculé pour chaque couple des sous-groupes d'apprentissage et de sélection, selon la formule suivante :

$$r(app, sel) = \frac{D_T - D\,(app\text{-}sel)}{D_T}$$

Dans laquelle,

R(app, sel) est un critère de représentativité entre les sous-groupes d'apprentissage et de sélection,
$D_T$ est la distance de MAHALANOBIS pour une loi de Fisher.
D(app, sel) est la distance théorique de MAHALANOBIS entre les deux sous-groupes d'apprentissage et de sélection.

R s'interprète de la manière suivante :

- si R = 0 les deux sous-groupes ne sont pas représentatifs l'un de l'autre,
- si R = 1 les deux sous-groupes sont confondus,
- plus la valeur de R s'approche de 1 plus les deux sous-groupes sont représentatifs l'un de l'autre,
- à l'inverse plus R est voisin de 0, moins les sous-groupes sont représentatifs l'un de l'autre.

Le critère P est déterminé par la formule suivante :

$$P(app, sel) = \left[ \sum_{j=1}^{k} S_{app}(j) \cdot S_{sel}(j) \right] \Big/ \sqrt{ \sum_{j=1}^{m} S_{app}^{2}(j) \cdot \sum_{j=1}^{m} S_{sel(j)}^{2} }$$

Dans laquelle :

- P(app, sel) est un critère d'homogénéité des sous-groupes d'apprentissage et de sélection,
- $S_{app}$ est la somme des vecteurs propres, pondérée par les valeurs propres du sous-groupe d'apprentissage,
- $S_{sel}$ est la somme des vecteurs propres, pondérée par les valeurs propres du sous-groupe de sélection,
- m est le nombre de variables égal à 6.
- k est le nombre de valeurs propres significatives des matrices de variance covariance de l'apprentissage et de la sélection,

P s'interprète de la manière suivante :

- si P = 0 les deux sous-groupes ne sont pas homogènes,
- si P = 1 les deux sous-groupes ont la même distribution dans l'espace,
- plus la valeur de P est voisine de 1 plus les sous-groupes sont homogènes,
- plus la valeur de P est voisine de 0 moins les sous-groupes sont homogènes,

P exprime la répartition spatiale des données en comparant les formes et les orientations des sous-groupes. On a déterminé expérimentalement que si P < 0,7 les deux sous-groupes ne sont pas homogènes.
Le critère C est déterminé par la formule suivante :

$$C(app, sel) = exp [- M(app, sel)/ [(n_{app}-1) + (n_{sel}-1)]]$$

dans laquelle :

$C(app, sel)$ est un critère d'homogénéité des sous-groupes d'apprentissage et de sélection,
$n_{app}$ est le nombre d'observations dans le sous-groupe d'apprentissage,
$n_{sel}$ est un nombre d'observations dans le sous-groupe de sélection,
$M(app, sel)$ est lui-même défini par la formule,

$$M(app, sel) = v \left[ (n_{app}-1) \cdot \log \left| A_{app}^{-1} \cdot A \right| + (n_{sel}-1) \log \left| A_{sel}^{-1} \cdot A \right| \right]$$

dans laquelle :
$|.|$ est un déterminant.

$$v = 1 - \frac{2p^2 + 3p-1}{6(p+1)} \left( \frac{1}{n_{app}-1} + \frac{1}{n_{sel}-1} - \frac{1}{n_{app}+n_{sel}-2} \right)$$

et dans laquelle :

- $p$ est le nombre de variables égal à 6.

  $A_{app}$ est la matrice de variance covariance du sous-groupe d'apprentissage.

- $A_{sel}$ est la matrice de variance covariance du sous-groupe de sélection.
- A est la matrice de variance covariance de l'ensemble des observations des deux sous-groupes d'apprentissage et de sélection.

  C s'interprète de la manière suivante :

- si $C = 0$ les covariances des sous-groupes d'apprentissage et de sélection sont différentes,
- si $C = 1$ les covariances des sous-groupes d'apprentissage et de sélection sont égales,
- plus la valeur de C est proche de 1 plus les covariances sont proches et les groupes homogènes,
- plus la valeur de C est proche de 0 moins les groupes sont homogènes.

Le critère global de qualité F est calculé de la manière suivante :

- si $P < 0,7$ alors $F = 0,1 P,$
- si $P \geq 0,7$ alors

$$F = \sqrt{(P^2 + C^2 + R^2)/3}$$

Une valeur de F voisine de 1 indique que les trois sous-groupes sont homogènes et représentatifs.
On choisit la répartition à modifier dans l'étape suivante, suivant une probabilité proportionnelle à la qualité de chacune des répartitions.
On génère une nouvelle répartition par modification de la répartition des données numériques dans les sous-groupes par une méthode qui met en oeuvre un algorithme génétique. Ce dernier est basé sur la représentation des trois sous-groupes en trois chromosomes composés d'une suite d'entiers correspondants au numéro d'ordre de l'observation dans le fichier initial.
On optimise les répartitions en appliquant alternativement des mutations et des reproductions.
On évalue la qualité de la nouvelle répartition à partir du critère F défini précédemment.
On réitère les trois étapes précédentes jusqu'à atteindre un nombre d'itérations de 1000.
On obtient alors un critère de qualité globale de la répartition, F égal à 0,98. Cette valeur très proche de 1 indique que les trois sous-groupes sont homogènes et représentatifs, et démontre l'intérêt du procédé de l'invention.

Si on calcule la valeur de F lorsqu'on utilise la méthode de KENNARD et STONE avec le même jeu de variables on obtient $F = 0,05$.

Cette valeur très faible de F, voisine de zéro, démontre que la méthode de KENNARD et STONE ne satisfait pas aux critères d'homogénéité et de représentativité souhaités.

La modélisation est réalisée par un réseau de neurones multicouches selon une méthode connue selon laquelle :

- le sous-groupe d'apprentissage sert à déterminer les poids de connexions du réseau de neurones,
- le sous-groupe de sélection sert à sélectionner la bonne architecture et les bons paramètres de l'algorithme,
- le sous-groupe de validation sert à calculer la capacité de généralisation.

L'adéquation du modèle à la réalité s'exprime par l'écart type de prédiction obtenu sur chacun des sous-groupes. On trouve :

- pour le sous-groupe d'apprentissage : 0,25 point d'indice d'octane,
- pour le sous-groupe de sélection : 0,30 point d'indice d'octane,
- pour le sous-groupe de validation : 0,25 point d'indice d'octane.

La répartition des sous-groupes satisfaisant aux critères d'homogénéité et de représentativité, le fait que ces trois valeurs soient proches l'une de l'autre montre que la modélisation est de bonne qualité.

Pour prédire la valeur de l'indice d'octane moteur de l'essence produite à un instant donné par le craqueur catalytique on utilise le modèle ainsi établi avec comme données d'entrée, les valeurs des six variables représentant les conditions opératoires et les caractéristiques de la charge, à l'instant considéré. La valeur de l'indice d'octane ainsi prédite peut être utilisée par l'opérateur pour conduire l'unité manuellement, ou comme donnée d'entrée d'un programme d'automatisation qui assure la conduite automatique de l'unité.

Le mode d'utilisation du procédé de l'invention qui vient d'être décrit est donné à titre d'exemple non limitatif. Le procédé de l'invention peut être utilisé pour prédire les valeurs de grandeurs physiques très diverses qui interviennent dans la conduite de procédés de fabrication, à partir d'un modèle et d'enregistrements de signaux délivrés par des instruments de mesure de paramètres physiques ou par des analyseurs, notamment des spectromètres infrarouges.

## Revendications

1. Procédé de détermination de la valeur d'une grandeur physique d'un produit à analyser à partir des valeurs d'enregistrements de signaux délivrés par au moins un instrument de mesure alimenté par ledit produit et d'un modèle validé, construit à partir d'un fichier initial de données numériques préalablement obtenues à partir des valeurs des enregistrements des signaux délivrés par l'instrument de mesure alimenté par une pluralité de produits et des valeurs connues de la grandeur physique de chacun desdits produits, caractérisé en ce qu'il consiste en outre à répartir les données numériques du fichier initial en sous-groupes homogènes et représentatifs, entre eux et par rapport audit fichier initial.

2. Procédé selon la revendication 1 caractérisé en ce qu'il comporte les étapes consistant à :

   - soumettre les données numériques du fichier initial à une transformation mathématique,
   - choisir un nombre de sous-groupes supérieur ou égal à deux,
   - choisir la taille de chacun desdits sous-groupes,
   - générer une ou plusieurs répartitions initiales des données numériques dans lesdits sous-groupes,
   - évaluer la qualité de la ou des répartitions initiales des données numériques en sous-groupes à partir d'au moins un critère de qualité,
   - choisir la répartition à modifier dans l'étape suivante dans le cas d'une pluralité de répartitions initiales,
   - générer une nouvelle répartition par modification de la répartition des données numériques dans les sousgroupes selon une technique d'optimisation,
   - évaluer la qualité de la nouvelle répartition à partir du ou des critères de qualité,
   - réitérer les trois étapes précédentes jusqu'à satisfaction d'un critère d'arrêt obtenu par comparaison de la valeur de la qualité de la dernière répartition à un seuil fixé ou jusqu'à ce que le nombre d'itérations atteigne un nombre maximal fixé.

3. Procédé selon la revendication 2 caractérisé en ce que le critère de qualité consiste en au moins un critère choisi parmi les suivants :

- critère de représentativité des données numériques des sous-groupes par rapport à la grandeur mesurée, qui met en oeuvre une métrique caractérisant une distance entre les sous-groupes,
- critère d'homogénéité servant à comparer entre elles d'une part les formes et d'autre part les orientations des sous-groupes,
- critère d'homogénéité servant à comparer entre elles les densités des données numériques dans les sous-groupes.

4. Procédé selon la revendication 2 ou 3 caractérisé en ce que la technique d'optimisation consiste en au moins une méthode choisie parmi les suivantes :

- méthode qui met en oeuvre au moins un algorithme génétique,
- méthode qui met en oeuvre au moins un réseau de neurones,
- méthode qui met en oeuvre au moins un algorithme de recuit simulé,
- méthode incrémentale,
- méthode qui met en oeuvre au moins un critère matriciel d'optimalité, et
- méthode itérative de type bootstrap.

5. Procédé selon l'une des revendications 2 à 4 caractérisé en ce que le choix de la taille des sous-groupes est optimisé par application d'une méthode d'optimisation itérative d'un critère de qualité.

**Office européen des brevets**

# RAPPORT DE RECHERCHE EUROPEENNE

Numero de la demande

EP 96 40 2908

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| X | PATENT ABSTRACTS OF JAPAN<br>vol. 95, no. 001<br>& JP 07 028770 A (MITSUBISHI ELECTRIC CORP), 31 Janvier 1995, | 1 | G01N33/28 |
| A | * abrégé *<br>--- | 2-5 | |
| A | US 5 360 972 A (DIFOGGIO ROCCO ET AL) 1 Novembre 1994<br>* abrégé; revendications 1,14; figures 1-4 *<br>----- | 1-5 | |

| | DOMAINES TECHNIQUES RECHERCHES (Int.Cl.6) |
|---|---|
| | G01N |

Le présent rapport a été établi pour toutes les revendications

| Lieu de la recherche | Date d'achèvement de la recherche | Examinateur |
|---|---|---|
| LA HAYE | 7 Avril 1997 | Chapple, I |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)